# EUROPÄISCHE PATENTANMELDUNG

(11) **EP 1 709 906 A1**
(43) Veröffentlichungstag der Anmeldung: **11.10.2006**
(21) Anmeldenummer: 05007580.3
(22) Anmeldetag: 07.04.2005
(51) Int. Cl.: A61B 5/151

(54) **Verfahren und Vorrichtung zur Entnahme von Körperflüssigkeit**

(71) Anmelder: F. HOFFMANN-LA ROCHE AG, 4070 Basel (CH); Roche Diagnostics GmbH, 68305 Mannheim (DE)
(72) Erfinder: Hein, Heinz-Michael, Dr., 6044 Udligenswil (CH); Abt, Redo, 5624 Bünzen (CH); Korner, Stephan, 6330 Cham (CH); Calasso, Irio, Dr., 6415 Arth (CH); Sarofim, Emad, 6300 Zug (CH); Griss, Patrick, Dr., 8057 Zürich (CH); Jaeggi, Rainer, Dr., 5610 Wohlen (CH)
(74) Vertreter: Pfiz, Thomas

(57) **Zusammenfassung**

Die Erfindung betrifft ein Verfahren und eine Vorrichtung zur Entnahme von Körperflüssigkeit, wobei ein Stechelement (10) in einer Vorwärtsbewegung (54) durch die Haut in ein Körperteil (14) eingestochen und die Körperflüssigkeit über eine Kapillarstruktur (18) des Stechelements (10) aufgenommen wird. Erfindungsgemäß wird vorgeschlagen, dass ein Körperflüssigkeitskontakt des Stechelements (10) nach der Vorwärtsbewegung (54) während einer Sammelphase (62) innerhalb des Körperteils (14) detektiert wird.

## Beschreibung

Die Erfindung betrifft ein Verfahren zur Entnahme von Körperflüssigkeit, insbesondere Blut, bei dem ein Stechelement in einer Vorwärtsbewegung durch die Haut in ein Körperteil eingestochen und die Körperflüssigkeit über eine Kapillarstruktur des Stechelements aufgenommen wird. Die Erfindung betrifft weiter eine entsprechende Vorrichtung.

Solche Verfahren und Entnahmevorrichtungen für kleine Körperflüssigkeitsmengen dienen vor allem Diabetikern zur täglich mehrfach durchgeführten Blutzucker-Selbstkontrolle. Neuere Konzepte sehen eine messende Mikronadel als Einwegartikel (Disposable) in einem Handgerät vor, um einen Hauteinstich zu erzeugen, daraus eine kleine Menge Blut unter Ausnutzung von Kapillarkräften zu entnehmen und diese Blutprobe zu analysieren. Mit einem solchen integrierten System sollen in einem weitgehend automatischen Messablauf auch von Laien die erforderlichen Schritte einfach und schnell vorgenommen werden können. Um dies zu erreichen, ist es wichtig, dass Stech- und Sammelvorgang geräteseitig auf das Disposable abgestimmt sind, damit effizient, schnell und schmerzfrei Blut oder gegebenenfalls Gewebeflüssigkeit gesammelt werden kann.

In diesem Zusammenhang wurde bereits vorgeschlagen, zur Steuerung der vorwärts gerichteten Einstechbewegung einen Hautkontakt zu erfassen, um so eine definierte Einstechtiefe erreichen zu können. Eine solche Bewegungssteuerung muss aber mit großem apparativem Aufwand an die gewünschte hohe Einstechgeschwindigkeit angepasst werden. Vor allem ist aber nachteilig, dass damit keine Erkenntnis über einen tatsächlichen Erfolg der Blutaufnahme gewonnen werden kann.

Zur Optimierung der Blutgewinnung beschreibt die WO 03/009759 A1 der Anmelderin ein Kombinationsverfahren, bei dem zunächst eingestochen wird, die Stecheinheit mit Kapillarstruktur sodann um einen Teil der Einstichstrecke zurückgezogen und dort für eine Sammelperiode von einigen Sekunden verharren gelassen wird. Dadurch soll ein Teil des Stichkanals freigegeben werden, so dass sich Körperflüssigkeit darin sammeln kann und von dort in die Kapillarstruktur eindringt.

Ausgehend hiervon liegt der Erfindung die Aufgabe zugrunde, die im Stand der Technik aufgetretenen Nachteile zu vermeiden und bei einfacher Benutzung eine insbesondere hinsichtlich Zuverlässigkeit, Effizienz, Schmerzminimierung und Hygiene optimierte Gewinnung der Körperflüssigkeit zu ermöglichen.

Zur Lösung dieser Aufgabe wird die in den unabhängigen Patentansprüchen jeweils angegebene Merkmalskombination vorgeschlagen.

Die Erfindung geht von dem Gedanken aus, anstelle einer Einstechkontrolle in einer nachgeschalteten Phase der Blutaufnahme eine Erfolgskontrolle zu ermöglichen. Demgemäß wird in verfahrensmäßiger Hinsicht vorgeschlagen, dass ein Körperflüssigkeitskontakt des Stechelements nach der Vorwärtsbewegung während einer Sammelphase innerhalb des Körperteils detektiert wird. Korrespondierend ist für eine Vorrichtung ein Detektionsmittel zur Erfassung eines Körperflüssigkeitskontakts des Stechelements nach der Vorwärtsbewegung während einer Sammelphase innerhalb des Körperteils vorgesehen. Auf diese Weise kann die Entnahme optimiert werden, da bei einem erfassten Flüssigkeitskontakt mit Sicherheit Körperflüssigkeit aus der Stichwunde nachgeflossen sein muss. Dies ermöglicht einen situationsgerechten Ablauf auch unter Berücksichtigung benutzerspezifischer Parameter wie lokaler Hautbeschaffenheit und Durchblutung. Die Zuverlässigkeit in Bezug auf die minimal notwendige Blutmenge für einen Test wird erhöht, und es ist eine schmerzminimierte Entnahme möglich, da die Dauer in Abhängigkeit von dem Ereignis des Flüssigkeitskontakts angepasst werden kann.

Ein weiterer Erfindungsaspekt liegt darin, den Sammelvorgang nicht statisch durchzuführen, sondern in einer schmerzarmen Zone einen verbesserten Blutzufluss zu gewährleisten. Dementsprechend wird ein vorgeschlagen, bei dem das Stechelement nach der Vorwärtsbewegung in einer Rückziehphase um einen ersten Weg und in einer späteren Sammelphase um einen zweiten Weg zurückgezogen wird, wobei die Rückziehphase kürzer als die Sammelphase und der erste Weg größer als der zweite Weg und der zweite Weg größer als Null ist. Eine entsprechende Vorrichtung sieht vor, dass der Stechantrieb zur Rückbewegung des Stechelements in einer Rückziehphase um einen ersten Weg und in einer späteren Sammelphase um einen zweiten Weg ausgebildet ist, wobei die Rückziehphase kürzer als die Sammelphase und der erste Weg größer als der zweite Weg und der zweite Weg größer als Null ist. Damit kann rasch und schmerzarm ein Stichkanal in einer Blutzone geschaffen werden, während der Sammelvorgang mit der erforderlichen Dauer für den Selbsttransport der Flüssigkeit in der Kapillarstruktur in einer unempfindlicheren äußeren Hautzone erfolgt. Überraschend hat sich gezeigt, dass durch das langsame Zurückziehen die Sammeleffizienz erheblich besser ist als bei statischen Sammelpositionen. Eine mögliche Erklärung kann darin liegen, dass die Haut nach der schnellen Stechbewegung durch das Stechorgan eingedrückt ist und diese Andruckkraft zunächst das Fließen des Blutes verhindert. Beim langsamen Zurückziehen während der Sammelphase wird jedoch die Haut wieder entspannt, so dass Blut besser Nachfließen kann. Denkbar ist es auch, dass durch das Zurückziehen des Stechelements eine Art Saugeffekt erreicht wird, so dass zusätzliches Blut zur Verfügung steht. Dabei ist das Sammeln im eingestochenen Zustand hygienisch günstig und für die dosierte Entnahme kleiner Flüssigkeitsmengen effizient.

Vorteilhafte Ausgestaltungen und Weiterbildungen der Erfindung ergeben sich aus den abhängigen Ansprüchen.

Durch die rasche Rückbewegung in eine Rückziehposition von geringerer Stechtiefe wird der Schmerzbereich des Körperteils bzw. der Haut schnell verlassen. In dieser äußeren nicht-blutgebenden Zone wird dann nach einer Wartezeit kontrolliert, ob Blut über den Stichkanal nachfliesst. Bei Flüssigkeitskontakt kann von einer hohen Sammelerfolgsrate ausgegangen werden, ohne überflüssiges Totvolumen. Außerdem kann noch in der Haut besonders hygienisch Flüssigkeit aufgenommen werden, ohne dass austretendes Blut zu sehen wäre. Zur Erfassung des Flüssigkeitskontakts eignet sich vor allem eine Impedanzmessung unter Einbeziehung des Stechorgans als Elektrode. Die Wahrscheinlichkeit für eine erfolgreiche Aufnahme der Flüssigkeit wird durch eine solche Kontrolle erheblich erhöht. Hierdurch kann auch die optimale Sammelzeit abgewartet werden. Gegebenenfalls wird bei ausbleibendem Kontakt ein Fehlersignal generiert und der Entnahmevorgang abgebrochen.

Besonders bevorzugt wird die Körperflüssigkeit nach dem schnellen Zurückziehen des Stechelements während einer Sammelphase in der äußeren schmerzarmen Hautschicht aufgenommen. Dabei wird das Stechelement zumindest während eines Teils der Sammelphase gleichförmig oder mit veränderlicher Geschwindigkeit zurückgezogen, wodurch die Sammeleffizienz entscheidend verbessert wird. Die Sammelzeit sollte ausreichend bemessen sein, um unter der kapillaraktiven Wirkung eine hinreichende Flüssigkeitsaufnahme sicherzustellen.

Insbesondere sind alle offenbarten Ausführungsformen hinsichtlich der Körperflüssigkeitserfassung und den Stechprofilen miteinander kombinierbar.

Im Folgenden wird die Erfindung anhand der in der Zeichnung schematisch dargestellten Ausführungsbeispiele näher erläutert. Es zeigen
- Fig. 1: ein Blockschaltbild einer Vorrichtung zur Entnahme und ggf. Analyse von Blut aus einem Körperteil;
- Fig. 2 bis 5: ein Stechelement der Vorrichtung nach Fig. 1 in verschiedenen Stechpositionen in vereinfachter Schnittdarstellung;
- Fig. 6 bis 9: verschiedene Stechprofile beim Einsatz des Stechelements; und
- Fig. 10: die Anatomie der menschlichen Haut in einer schaubildlichen Darstellung.

Die in der Zeichnung dargestellte Vorrichtung dient der Selbstentnahme einer Blutprobe durch einen Benutzer für Analysezwecke, insbesondere zur Blutzuckerkontrolle. Die Vorrichtung umfasst ein Stechelement 10 als Einmalartikel zur Blutaufnahme und ein Handgerät 12 zur automatischen Handhabung des eingesetzten Stechelements 10.

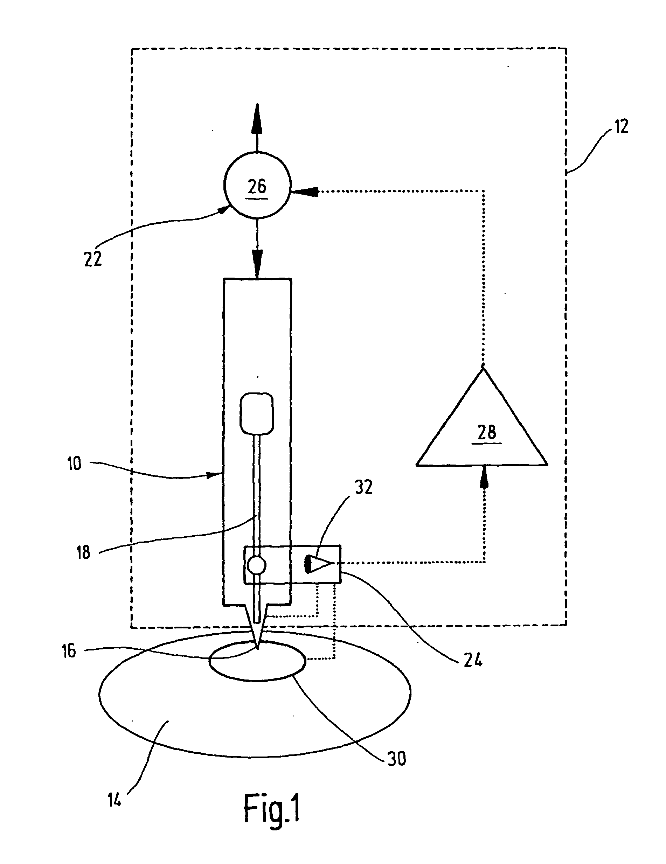

Das in Fig. 1 gezeigte Stechelement 10 ist als so genannter "Microsampler" zur Gewinnung einer kleinen Blutmenge aus einem Körperteil 14, insbesondere einer Fingerkuppe vorgesehen. Es besteht als Flachformteil aus dünnem Edelstahlblech und weist eine distal angeformte Spitze 16 als Stechorgan auf, die über einen halboffenen rillenförmigen Kapillarkanal 18 mit einer Sammelstelle 20 verbunden ist, welche als Reaktionsbereich für einen Nachweis eines Analyten, z. B. Glucose ausgebildet sein kann. Der Blutglucosenachweis speziell mittels berührungsloser optischer Methoden ist im Stand der Technik an sich bekannt und wird daher hier nicht näher erläutert. Optional kann das gesammelte Blut aber auch in eine Analyseeinheit transferiert werden, um dort den Analyten zu bestimmen.

Das Gerät bzw. Instrument 12 weist einen Stechantrieb 22 für eine kontrollierte Vorwärts- und Rückbewegung des Stechelements 10 und Detektionsmittel 24 zur Erfassung eines Blutkontakts des Stechelements 10 während einer Sammelphase auf. Der Stechantrieb 22 umfasst eine mechanisch und/oder elektrisch arbeitende Antriebseinheit 26, die mit dem Stechelement 10 gekoppelt ist. Speziell kann ein zweistufiger Hybridantrieb vorgesehen sein, bei dem eine schnelle Bewegung von einer Mechanik, beispielsweise einer Antriebsfeder oder Antriebskulisse übernommen wird, während ein langsamere geregelte Bewegung über einen Elektromotor erfolgt. Alternativ kann auch ein elektromagnetischer Antrieb nach Art einer Lautsprecherspule den Stechhub bewirken. Der Stechantrieb 22 umfasst ferner eine Steuereinheit 28 für die Ablaufsteuerung oder -regelung der Blutentnahme und insbesondere der Stechbewegung. Zweckmäßig wird hierfür ein Mikrocontroller eingesetzt, der gegebenenfalls zusätzlich benutzerspezifisch parametrisiert werden kann.

Die Detektionsmittel 24 können den Blutkontakt während der Sammelphase durch eine Impedanzmessung über das Stechorgan 16 und einen von dem Stechorgan axial durchsetzten und in Anlage mit dem Körperteil 14 befindlichen Andruckring 30 als Gegenelektrode erfassen. Das aus elektrisch leitfähigem Material bestehende Stechorgan kann hierbei mit einem elektrischen Wechselspannungssignal, z.B. 4 Vpp und 10 kHz gefahrlos angesteuert werden, wobei durch einen fluidischen Kontakt die Impedanz in dem Pfad zwischen Stechorgan und Gegenelektrode signifikant abnimmt. Eine Kontrolleinrichtung 32 ermöglicht eine Erfolgskontrolle der Blutaufnahme in Abhängigkeit von dem erfassten Flüssigkeitskontakt, wie es nachstehend näher erläutert wird.

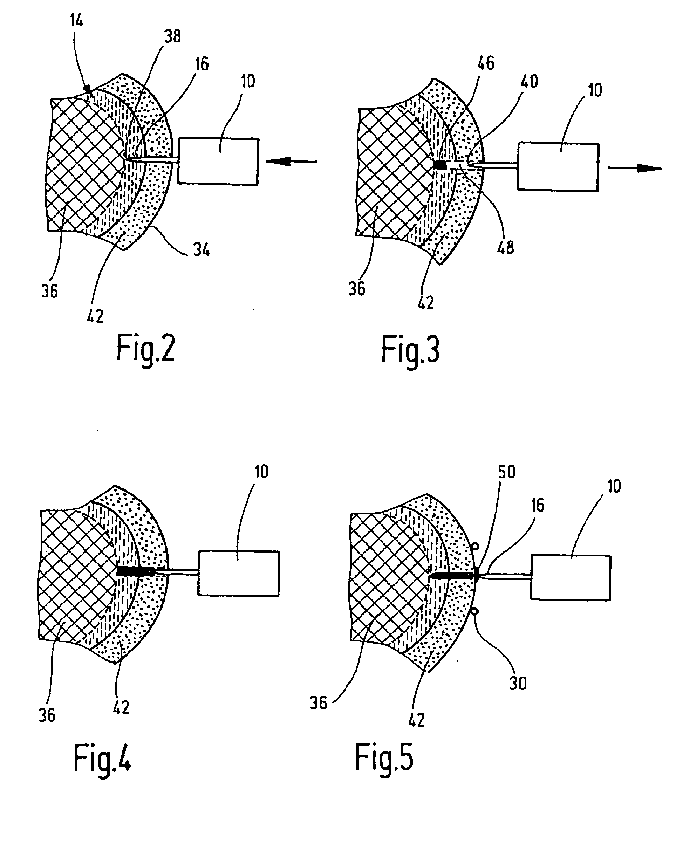

Die Fig. 2 bis 5 veranschaulichen den Ablauf der Blutgewinnung mittels des Stechelements 10. Zunächst wird in einer distal gerichteten Vorwärtsbewegung das Stechorgan 16 durch die Hautoberfläche 34 hindurch in das Körperteil 14 auf eine vorgegebene Tiefe eingestochen, so dass eine blutgebende Zone 36 in der Dermis erreicht wird. In diesem Bereich enden die Blutkapillaren, die durch das Stechorgan geöffnet werden können. Zugleich ist aber in diesem tieferen Bereich 36 aufgrund der dort vorhandenen Nervenzellen auch das Schmerzempfinden hoch. Aus diesem Grund ist es günstig, wenn das Stechelement unmittelbar nach der Vorwärtsbewegung in einer Rückziehbewegung aus der tiefsten Einstechposition 38 in eine Rückziehposition 40 von geringerer Stechtiefe zurückgezogen wird (Fig. 3). Bevorzugt liegt diese Rückziehposition im Bereich der Epidermis 42, speziell im Stratum Corneum 44. Nach dem schnellen Zurückziehen des Stechelements 10 verstreicht eine gewisse Zeit, bis das Blut 46 über den erzeugten Stichkanal 48 in die Epidermis nachfliest.

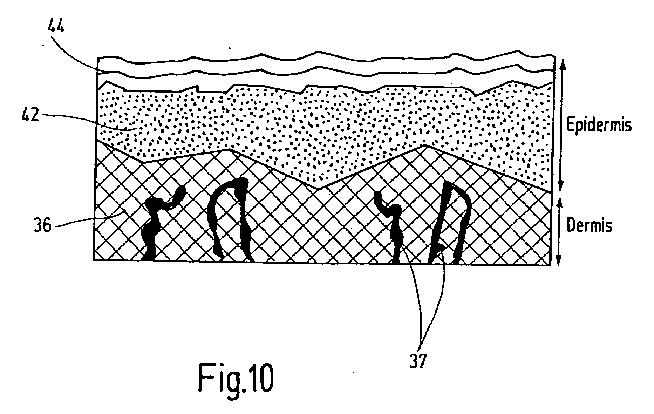

Die Dimensionen des Schichtaufbaus der menschlichen Haut sind in Fig. 10 realistischer veranschaulicht. Die Epidermis mit dem Stratum Corneum als oberster Hautschicht umfasst etwa 0,5 bis 1 mm Tiefe, während die Blutkapillaren 37 enthaltende Dermis in einen Tiefenbereich von einigen Millimetern erreicht.

Gemäß Fig. 4 wird in der zurückgezogenen Sammelposition 40 der Blutkontakt nach einer Wartezeit nach der Vorwärtsbewegung durch die Detektionsmittel 24 im Sinne einer Erfolgskontrolle erfasst. Auf diese Weise kann die Sammelzeit optimiert werden, da bei einem Flüssigkeitskontakt mit Sicherheit Blut aus der Stichwunde nachgeflossen ist. Wenn hingegen nach der Wartezeit kein Blutkontakt detektiert wurde, kann die Messung durch die Kontrolleinrichtung 32 abgebrochen werden. Dadurch entstehen keine falschen Resultate durch fehlendes oder zu kleines Blutvolumen. Zweckmäßig gibt die Kontrolleinrichtung 32 eine entsprechende Meldung an den Benutzer aus.

Der Sammelvorgang braucht nicht statisch zu erfolgen, sondern kann im Zuge einer weiteren Rückbewegung des Stechelements 10 durchgeführt werden. Optional ist auch ein Sammeln auf der Hautoberfläche 34 möglich, wie es in Fig. 5 gezeigt ist. Hierbei kann ein Flüssigkeitskontakt durch das Stechorgan 16 als Messelektrode, welche in einen ausgetretenen Blutstropfen 50 eintaucht und damit in fluidischem Kontakt mit der Haut steht, und dem an die Haut anliegenden Andruckring 30 als Gegenelektrode zuverlässig erfasst werden. Der Andruckring ist zusätzlich dafür vorgesehen, den Blutfluss unter Druck zu unterstützen und die Wunde zu öffnen, so dass der Kontakt zwischen Stechorgan und Haut und damit der Schmerz für den Benutzer minimal ist.

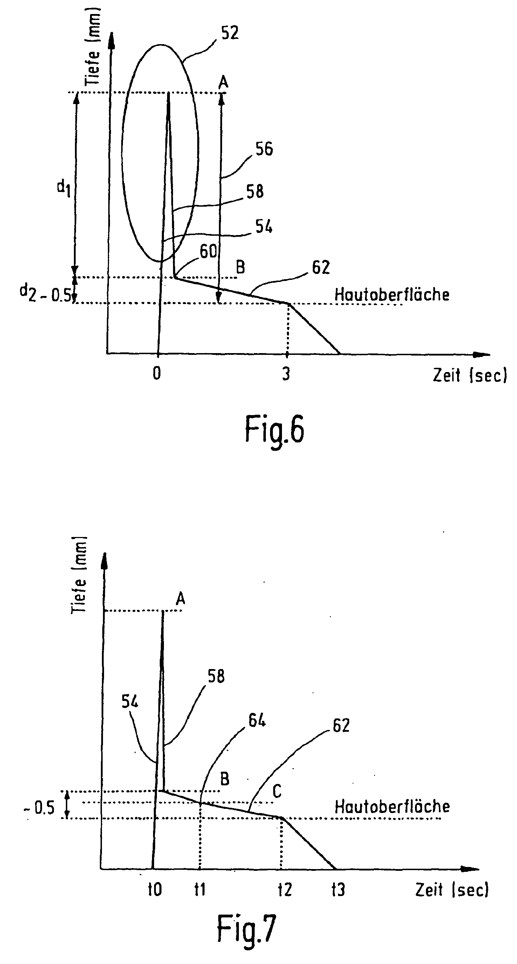

Für eine möglichst erfolgreiche und schmerzarme Blutaufnahme sind die in den Fig. 6 bis 9 gezeigten Stechprofile besonders vorteilhaft. Unter dem Begriff "Stechprofil" ist hierbei der Zeitverlauf der Stechbewegung zu verstehen, wie er als Funktion der Stechtiefe über der Zeit dargestellt ist.

Bei dem in Fig. 6 gezeigten Stechprofil wird das Stechorgan in den blutgebenden, schmerzempfindlichen Hautbereich 52 in einer Vorwärtsbewegung 54 bis zu einer vorgegebenen Tiefe 56 schnell eingestochen und sogleich in einer Rückziehbewegung 58 bis auf eine Tiefe von ca. 0,5 mm zurückgezogen. Die Rückziehphase liegt dabei in der Größenordnung der Dauer der Vorwärtsbewegung, d. h. wenigen 100 µs. Die Rückziehposition 60 kann sich im Bereich des Stratum Corneums und damit außerhalb des Schmerzbereichs 52 befinden. Anschließend folgt eine Sammelphase 62, in welcher Blut aufgenommen wird, während das Stechelement langsam bis auf die Hautoberfläche zurückgezogen wird. Diese Sammelphase 62 dauert einige Sekunden, wobei der durch das Stechorgan zurückgelegte Weg d2 wesentlich kleiner ist als der Rückziehweg d1 während der Rückziehphase 58. Durch das spätere langsamere Zurückziehen relaxiert die Haut, so dass der Stichkanal nicht sogleich wieder blockiert wird.

Da der Sammelvorgang eine gewisse Zeit benötigt, wird er zweckmäßigerweise in der nervenfreien äußeren Hautzone vollzogen. Das Sammeln unterhalb der Hautoberfläche, d.h. im eingestochenen Zustand, ist ebenfalls wichtig, um einen Blutaustritt auf die Haut zu vermeiden und somit einen besonders hygienischen Entnahmevorgang zu ermöglichen. Hinzu kommt, dass bereits auf die Hautoberfläche ausgetretenes Blut nur dann sauber mit dem Stechelement 10 entfernt werden kann, wenn die Kapillarkraft der Kapillare 18 an dieser Stelle genügend groß ist. Dies ist dann der Fall, wenn das Stechorgan 16 sich zumindest in leicht eingestochenem Zustand befindet, weil zu der Spitze hin die Kapillartiefe kleiner wird und damit die Kapillarkraft auch abnimmt.

Für das Stechprofil nach Fig. 6 sind elektrische oder elektromagnetische Antriebe besser geeignet als rein mechanische Antriebe, weil bei letzteren Prelleffekte schwerer zu kontrollieren sind (eine Dämpfung lässt sich einfacher elektrischer realisieren als durch eine mechanische Lösung).

Um die Stechtiefe definiert einstellen zu können, kann eine Detektion der Hautoberfläche beispielsweise durch eine Impedanzmessung vor dem eigentlichen Stechvorgang vorgenommen werden. Die Position der Hautoberfläche wird dabei während einer langsamen Vorwärtsbewegung des Stechelements 10 detektiert.

Das Umschalten zwischen den verschiedenen Bereichen des Stechprofils kann entweder durch die Position (Tiefe) gesteuert werden oder zeitgesteuert sein. Der Übergang zwischen den Bereichen mit unterschiedlichen Geschwindigkeiten kann durch eine unstetige Änderung erfolgen oder aber fließend sein (stetige Geschwindigkeitsänderung). Die stetige Lösung hat den Vorteil, dass weniger Antriebsenergie erforderlich ist und die Regelung der Bewegung vereinfacht wird.

Das in Fig. 7 gezeigte Stechprofil unterscheidet sich nur durch eine zusätzliche Stufe 64 während der Sammelphase 62. Hier erfolgt nach dem schnellen Zurückziehen bis Tiefe B ein etwas langsameres Zurückziehen bis Tiefe C, wobei sich während dieses Intervalls die Haut entspannt. Der eigentliche Sammelvorgang erfolgt dann nochmals verlangsamt von der Tiefe C bis an die Hautoberfläche. Dadurch kann eine Verkürzung des gesamten Stech- und Sammelvorgangs erreicht werden.

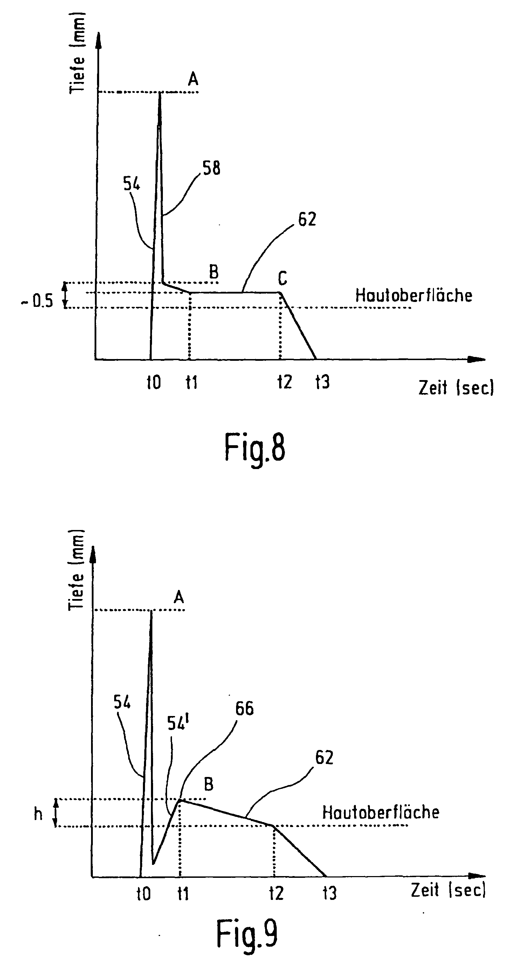

Eine ähnliche Ausführung sieht gemäß Fig. 8 vor, dass nach der schnellen Rückziehphase 58 die Hautentspannung durch langsames Zurückziehen des Stechorgans bis zum Zeitpunkt t1 erreicht wird, und anschließend bis zum Zeitpunkt t2 bei ruhendem Stechorgan weiter gesammelt wird. Hierbei ist allerdings eine seitliche Verschiebung des Stechorgans gegenüber der Haut für den Benutzer deutlicher zu spüren.

Bei dem Stechprofil nach Fig. 9 wird das Stechorgan nach einer ersten schnellen Vorwärtsbewegung 54 vollständig aus der Haut zurückgezogen und anschließend in einer zweiten Vorwärtsbewegung 54' mit geringerer Stechtiefe erneut eingestochen. In diesem Punkt wird dann ein Blutkontakt detektiert, und anschließend der Sammelvorgang 62 unter langsamem Zurückziehen des Stechorgans bis zur Hautoberfläche durchgeführt. Das vollständige Zurückziehen besitzt den Vorteil, dass ein eventuelles Nachprellen des Stechorgans außerhalb der Haut stattfindet und somit keine Dämpfung nötig ist, was die Anforderungen an die Aktuatorik stark reduziert.

Dieses Stechprofil bietet sich für eine einfache Regelungsvariante an. Zunächst kann vor der Zeit t0 die Hautoberfläche detektiert werden, um die Stechtiefe genau bestimmen zu können. Dieser Vorgang könnte induktiv oder durch eine Impedanzmessung erfolgen, indem wie oben beschrieben das Stechelement langsam gegen die Haut bewegt wird, bis deren Oberfläche angetastet wird. Der erste Stechvorgang (schnell hinein und schnell heraus) kann dann gleichsam "blind" erfolgen, d.h. ohne Rückkopplung durch einen Regelkreis. Dies hat den Vorteil, dass insbesondere im Falle der Verwendung eines elektromagnetischen Spulenantriebs eine schnelle und kostspielige Regelungselektronik vermieden werden kann. Bei einem Hybridantrieb würde dieser schnelle Stechvorgang vom mechanischen Teil übernommen (z. B. Feder- oder Kulissen-Antrieb). Nach dem ersten Stechvorgang wird das Stechorgan langsamer vorwärts bewegt als zuvor (Phase 54'). Diese Bewegung kann ebenfalls ohne Regelschleife erfolgen. Ist die Hautoberfläche erreicht (ggf. erfasst durch erneute Hautdetektion), so kann ein Steuersignal ausgelöst werden, welches den verbleibenden Abschnitt des Stechprofils (Sammelphase) triggert. Der Umkehrpunkt 66 kann gegenüber dem Triggersignal durch eine zeitliche Verzögerung bestimmt werden. Diese zeitliche Verzögerung muss kurz genug sein, um nicht wieder bis in die Schmerzzone 52 vorzudringen. Der Sammelvorgang bis t2 kann dann ebenfalls ohne Positionsregelung durch einfache Steuerung erfolgen. Anschließend wird das Stechelement bis zum Zeitpunkt t3 in das Gerät zurückgezogen und das Stechorgan somit von der Haut entfernt.

## Patentansprüche

1. Verfahren zur Entnahme von Körperflüssigkeit, insbesondere Blut, bei dem ein Stechelement (10) in einer Vorwärtsbewegung (54) durch die Haut in ein Körperteil (14) eingestochen und die Körperflüssigkeit über eine Kapillarstruktur (18) des Stechelements (10) aufgenommen wird, **dadurch gekennzeichnet, dass** ein Körperflüssigkeitskontakt des Stechelements (10) nach der Vorwärtsbewegung (54) während einer Sammelphase (62) innerhalb des Körperteils (14) detektiert wird.

2. Verfahren nach Anspruch 1, **dadurch gekennzeichnet, dass** das Stechelement (10) nach der Vorwärtsbewegung (54) in einer Rückziehbewegung aus der tiefsten Einstechposition in eine Rückziehposition (60) von geringerer Stechtiefe zurückgezogen wird.

3. Verfahren nach Anspruch 2, **dadurch gekennzeichnet, dass** der Körperflüssigkeitskontakt in der Rückziehposition (60) erfasst wird.

4. Verfahren nach einem der Ansprüche 1 bis 3, **dadurch gekennzeichnet, dass** der Körperflüssigkeitskontakt nach einer Wartezeit nach der Vorwärtsbewegung (54) erfasst wird.

5. Verfahren nach einem der Ansprüche 1 bis 4, **dadurch gekennzeichnet, dass** das Stechelement (10) nach einer ersten Vorwärtsbewegung (54) aus der Haut zurückgezogen und sogleich in einer zweiten Vorwärtsbewegung (54') mit geringerer Stechtiefe erneut eingestochen wird, und dass anschließend ein Körperflüssigkeitskontakt detektiert wird.

6. Verfahren nach Anspruch 5, **dadurch gekennzeichnet, dass** der Körperflüssigkeitskontakt beim erneuten Einstechen im Umkehrpunkt (66) zu einer Rückziehbewegung erfasst wird.

7. Verfahren nach einem der Ansprüche 1 bis 6, **dadurch gekennzeichnet, dass** der Körperflüssigkeitskontakt im Bereich einer äußeren Hautschicht (42), vorzugsweise im Bereich des Stratum Corneums (44) erfasst wird.

8. Verfahren nach einem der Ansprüche 1 bis 7, **dadurch gekennzeichnet, dass** die Körperflüssigkeit in einer nicht oder schwach mit Blut versorgten Hautschicht des Körperteils (14) aufgenommen wird.

9. Verfahren nach einem der Ansprüche 1 bis 8, **dadurch gekennzeichnet, dass** die Körperflüssigkeit über den beim Zurückziehen des Stechelements (10) freigegebenen Stichkanal (48) als Verbindung zu einer blutgebenden Zone (36) des Körperteils (14) aufgenommen wird.

10. Verfahren nach einem der Ansprüche 1 bis 9, **dadurch gekennzeichnet, dass** zumindest eine Teilmenge der Körperflüssigkeit auf der Haut außerhalb des Körperteils (14) aufgenommen wird.

11. Verfahren nach einem der Ansprüche 1 bis 10, **dadurch gekennzeichnet, dass** der Körperflüssigkeitskontakt über das Stechelement (10) und ein in Anlage mit dem Körperteil (14) stehendes Kontaktelement (30) detektiert wird.

12. Verfahren nach einem der Ansprüche 1 bis 11, **dadurch gekennzeichnet, dass** der Körperflüssigkeitskontakt durch einen elektrischen Messparameter insbesondere kapazitiv oder induktiv oder resistiv erfasst wird.

13. Verfahren nach einem der Ansprüche 1 bis 12, **dadurch gekennzeichnet, dass** der Körperflüssigkeitskontakt über das Stechelement (10) durch eine Impedanzmessung gegenüber der Haut erfasst wird.

14. Verfahren nach einem der Ansprüche 1 bis 11, **dadurch gekennzeichnet, dass** der Körperflüssigkeitskontakt vorzugsweise durch die Kapillarstruktur (18) oder einen Lichtleiter hindurch innerhalb des Körperteils (14) optisch erfasst wird.

15. Verfahren nach einem der Ansprüche 1 bis 14, **dadurch gekennzeichnet, dass** die erfolgreiche Aufnahme einer Probe der Körperflüssigkeit über die Detektion des Körperflüssigkeitskontakts kontrolliert wird.

16. Verfahren nach einem der Ansprüche 1 bis 15, **dadurch gekennzeichnet, dass** während der Erfassung des Körperflüssigkeitskontakts eine vorgegebene Sammeldauer für die Aufnahme der Körperflüssigkeit abgewartet und anschließend das Stechelement (10) vollständig aus dem Körperteil (14) zurückgezogen wird.

17. Verfahren nach einem der Ansprüche 1 bis 16, **dadurch gekennzeichnet, dass** bei ausbleibendem Körperflüssigkeitskontakt ein Fehlersignal generiert und der Entnahmevorgang abgebrochen wird.

18. Verfahren zur Entnahme von Körperflüssigkeit, insbesondere Blut, bei dem ein Stechelement (10) in einer Vorwärtsbewegung (54) durch die Haut in ein Körperteil (14) eingestochen und die Körperflüssigkeit über eine Kapillarstruktur (18) des Stechelements (10) aufgenommen wird, **dadurch gekennzeichnet, dass** das Stechelement (10) nach der Vorwärtsbewegung (54) in einer Rückziehphase (58) um einen ersten Weg (d1) und in einer späteren Sammelphase (62) um einen zweiten Weg (d2) zurückgezogen wird, wobei die Rückziehphase (58) kürzer als die Sammelphase (62) und der erste Weg (d1) größer als der zweite Weg (d2) und der zweite Weg größer als Null ist.

19. Verfahren nach einem der Ansprüche 1 bis 18, **dadurch gekennzeichnet, dass** die Körperflüssigkeit im Wesentlichen während der Sammelphase (62) aufgenommen wird.

20. Verfahren nach Anspruch 18 oder 19, **dadurch gekennzeichnet, dass** die Dauer der Rückziehphase (58) in der Größenordnung der Dauer der Vorwärtsbewegung (54) gewählt wird.

21. Verfahren nach einem der Ansprüche 18 bis 20, **dadurch gekennzeichnet, dass** die Sammelphase (62) gegenüber der Rückziehphase (58) um das 10- bis 10.000-fache verlängert wird.

22. Verfahren nach einem der Ansprüche 1 bis 21, **dadurch gekennzeichnet, dass** das Stechelement (10) während der Sammelphase (62) vorzugsweise mit gleichförmiger Geschwindigkeit zurückgezogen wird.

23. Verfahren nach einem der Ansprüche 1 bis 21, **dadurch gekennzeichnet, dass** das Stechelement (10) in einem ersten Zeitabschnitt der Sammelphase (62) gegenüber einem nachfolgenden zweiten Zeitabschnitt schneller zurückgezogen wird.

24. Verfahren nach Anspruch 23, **dadurch gekennzeichnet, dass** das Stechelement (10) in dem zweiten Zeitabschnitt für eine bestimmte Verweilzeit angehalten wird.

25. Verfahren nach einem der Ansprüche 18 bis 24, **dadurch gekennzeichnet, dass** das Stechelement (10) in der Rückziehphase (58) vollständig aus der Haut zurückgezogen und vor der Sammelphase (62) mit geringerer Stechtiefe erneut eingestochen wird.

26. Verfahren nach einem der Ansprüche 18 bis 25, **dadurch gekennzeichnet, dass** das Stechelement (10) während der Sammelphase (62) um einen zweiten Weg zwischen 2 mm und 0,1 mm zurückbewegt wird.

27. Verfahren nach einem der Ansprüche 1 bis 26, **dadurch gekennzeichnet, dass** das Stechelement (10) während der Sammelphase (62) im Bereich des Stratum Corneums (44) zurückgezogen wird.

28. Verfahren nach einem der Ansprüche 1 bis 27, **dadurch gekennzeichnet, dass** die Bewegung des Stechelements (10) durch eine Zeiterfassung oder eine Positionserfassung kontrolliert wird.

29. Vorrichtung zur Entnahme von Körperflüssigkeit, insbesondere Blut, mit einem eine Kapillarstruktur (18) zur Aufnahme der Körperflüssigkeit aufweisenden Stechelement (10) zum Einstechen durch die Haut in ein Körperteil (14) und einem Stechantrieb (22) für eine Vorwärts- und Rückbewegung des Stechelements (10), **gekennzeichnet durch** ein Detektionsmittel (24) zur Erfassung eines Körperflüssigkeitskontakts des Stechelements (10) nach der Vorwärtsbewegung (54) während einer Sammelphase (62) innerhalb des Körperteils (14).

30. Vorrichtung nach Anspruch 29, **dadurch gekennzeichnet, dass** das Stechelement (10) mittels des Stechantriebs (22) nach der Vorwärtsbewegung (54) in einer Rückziehbewegung aus der tiefsten Einstechposition in eine Rückziehposition von geringerer Stechtiefe zurückziehbar ist, und dass das Detektionsmittel (24) in der Rückziehposition bei einem Körperflüssigkeitskontakt anspricht.

31. Vorrichtung nach Anspruch 29 oder 30, **dadurch gekennzeichnet, dass** der Körperflüssigkeitskontakt nach Ablauf einer mittels eines Zeitgebers bestimmten Wartezeit nach der Vorwärtsbewegung (54) erfassbar ist.

32. Vorrichtung nach einem der Ansprüche 29 bis 31, **dadurch gekennzeichnet, dass** der Stechantrieb (22) dazu ausgebildet ist, das Stechelement (10) nach einer ersten Vorwärtsbewegung (54) aus der Haut zurückzuziehen und sogleich in einer zweiten Vorwärtsbewegung (54) mit geringerer Stechtiefe erneut einzustechen, und dass anschließend das Detektionsmittel (24) bei einem Körperflüssigkeitskontakt anspricht.

33. Vorrichtung nach einem der Ansprüche 29 bis 32, **dadurch gekennzeichnet, dass** der Stechantrieb (22) zweistufig ausgebildet ist, wobei eine vorzugsweise mechanisch arbeitende erste Antriebsstufe für eine schnelle Bewegung und eine vorzugsweise elektrisch arbeitende zweite Antriebsstufe für eine langsamere geregelte Bewegung des Stechelements (10) vorgesehen ist.

34. Vorrichtung nach einem der Ansprüche 29 bis 33, **dadurch gekennzeichnet, dass** der Körperflüssigkeitskontakt über das Stechelement (10) und ein in Anlage mit dem Körperteil (14) stehendes Kontaktelement (30) detektierbar ist.

35. Vorrichtung zur Entnahme von Körperflüssigkeit, insbesondere Blut, mit einem eine Kapillarstruktur (18) zur Aufnahme der Körperflüssigkeit aufweisenden Stechelement (10) zum Einstechen durch die Haut in ein Körperteil (14) und einem Stechantrieb (22) für eine Vorwärts- und Rückbewegung des Stechelements (10), **gekennzeichnet durch** Elektroden (10,30) als Detektionsmittel zur Erfassung eines Körperflüssigkeitskontakts des Stechelements (10) auf der Haut außerhalb des Körperteils (14), wobei die Elektroden **durch** das über die Körperflüssigkeit in fluidischen Kontakt mit der Haut befindliche Stechelement (10) und ein in Anlage mit der Haut stehendes Kontaktelement (30) gebildet sind.

36. Vorrichtung nach einem der Ansprüche 29 bis 35, **dadurch gekennzeichnet, dass** die Detektionsmittel (10,30;24) den Körperflüssigkeitskontakt durch einen elektrischen Messparameter insbesondere kapazitiv oder induktiv oder resistiv erfassen.

37. Vorrichtung nach einem der Ansprüche 29 bis 36, **dadurch gekennzeichnet, dass** der Körperflüssigkeitskontakt über das Stechelement (10) durch eine Impedanzmessung gegenüber der Haut erfassbar ist.

38. Vorrichtung nach einem der Ansprüche 29 bis 33, **dadurch gekennzeichnet, dass** der Körperflüssigkeitskontakt über optische Detektionsmittel vorzugsweise durch die Kapillarstruktur (18) oder einen Lichtleiter hindurch innerhalb des Körperteils (14) erfassbar ist.

39. Vorrichtung nach einem der Ansprüche 29 bis 38, **gekennzeichnet durch** eine Kontrolleinrichtung (32) zur Erfolgskontrolle der Flüssigkeitsaufnahme nach Maßgabe des detektierten Körperflüssigkeitskontakts.

40. Vorrichtung nach Anspruch 39, **dadurch gekennzeichnet, dass** die Kontrolleinrichtung (32) bei ausbleibendem Körperflüssigkeitskontakt ein Fehlersignal generiert und den Entnahmevorgang abbricht.

41. Vorrichtung zur Entnahme von Körperflüssigkeit, insbesondere Blut, mit einem eine Kapillarstruktur (18) zur Aufnahme der Körperflüssigkeit aufweisenden Stechelement (10) zum Einstechen durch die Haut in ein Körperteil (14) und einem Stechantrieb (22) für eine Vorwärts- und Rückbewegung des Stechelements (10), **dadurch gekennzeichnet, dass** der Stechantrieb (22) zur Rückbewegung des Stechelements (10) in einer Rückziehphase (58) um einen ersten Weg (d1) und in einer späteren Sammelphase (62) um einen zweiten Weg (d2) ausgebildet ist, wobei die Rückziehphase (58) kürzer als die Sammelphase (62) und der erste Weg (d1) größer als der zweite Weg (d2) und der zweite Weg größer als Null ist.

42. Vorrichtung nach Anspruch 41, **dadurch gekennzeichnet, dass** die Rückziehphase (58) in der Größenordnung der Dauer der Vorwärtsbewegung (54) liegt.

43. Vorrichtung nach Anspruch 41 oder 42, **dadurch gekennzeichnet, dass** die Sammelphase (62) gegenüber der Rückziehphase (58) um das 10- bis 10.000-fache verlängert ist.

44. Vorrichtung nach einem der Ansprüche 29 bis 43, **dadurch gekennzeichnet, dass** der Stechantrieb (22) das Stechelement (10) während der Sammelphase (62) vorzugsweise mit gleichförmiger Geschwindigkeit zurückzieht.

45. Vorrichtung nach einem der Ansprüche 29 bis 43, **dadurch gekennzeichnet, dass** der Stechantrieb (22) das Stechelement (10) in einem ersten Zeitabschnitt der Sammelphase (62) gegenüber einem nachfolgenden zweiten Zeitabschnitt schneller zurückzieht.

46. Vorrichtung nach einem der Ansprüche 29 bis 43, **dadurch gekennzeichnet, dass** der Stechantrieb (22) das Stechelement (10) in dem zweiten Zeitabschnitt für eine bestimmte Verweilzeit anhält.

47. Vorrichtung nach einem der Ansprüche 41 bis 46, **dadurch gekennzeichnet, dass** der Stechantrieb (22) das Stechelement (10) während der Rückziehphase (58) vollständig aus der Haut zurückzieht und vor der Sammelphase (62) mit geringerer Stechtiefe erneut einsticht.

48. Vorrichtung nach einem der Ansprüche 41 bis 47, **dadurch gekennzeichnet, dass** der Stechantrieb (22) das Stechelement (10) während der Sammelphase (62) um einen zweiten Weg zwischen 2 mm und 0,1 mm zurückbewegt.

49. Vorrichtung nach einem der Ansprüche 29 bis 48, **dadurch gekennzeichnet, dass** die Bewegung des Stechelements (10) durch eine Zeit- oder Positionserfassung gesteuert ist.
